# EUROPEAN PATENT APPLICATION

(11) **EP 2 514 814 A1**
(43) Date of publication of application: **24.10.2012**
(21) Application number: 10836976.0
(22) Date of filing: 14.09.2010
(51) Int. Cl.: C12M 1/107, C12M 1/02, C12P 5/02

(54) **CIRCULATION APPARATUS FOR COMPLETE MIX ANAEROBIC REACTION AND METHOD FOR CIRCULATION OF BIOGAS REACTION FEEDSTOCK**

(30) Priority: 17.12.2009 CN 200910259272
(71) Applicant: China Southern Airlines Industry (Group) Co., Ltd., Hunan 412002 (CN)
(72) Inventor: WANG, Guolin, Zhuzhou Hunan 412002 (CN); XIONG, Yijun, Zhuzhou Hunan 412002 (CN); HUANG, Wei, Zhuzhou Hunan 412002 (CN); JIANG, Yong, Sichuan 610041 (CN); WANG, Wei, Zhuzhou Hunan 412002 (CN); LEI, Chengming, Zhuzhou Hunan 412002 (CN)
(74) Representative: Bongiovanni, Simone
(86) International application number: PCT/CN2010/076910
(87) International publication number: WO 2011/072539

(57) **Abstract**

The present invention discloses a total mixed anaerobic reaction circulation device for methane, comprising a total mixed anaerobic reaction tank and a circulation tank, wherein the top portion of the total mixed anaerobic reaction tank is connected to the circulation tank via the overflow pipe; the lower portion of the circulation tank is connected to the lower portion of the anaerobic reaction tank via the transporting pipe and the material transporting pump; and the circulation tank is connected to the transporting pipe that supplies the methane reaction materials. The circulation tank is provided with the heat exchanger and the central cylinder therein; the overflow pipe is connected to the central cylinder; and the lower end of the central cylinder has the outlet pipe. The present invention also provides a circulation method of the methane reaction material. In the present invention, the use of the circulation tank simultaneously takes into account the functions such as addition and stirring of the methane reaction materials, heating of the methane reaction materials, adjustment of PH value, methane liquid recovery, etc. The total mixed anaerobic reaction circulation device for methane of the present invention is suitable for the large-scale industrialized methane project and can be popularized and used in cold areas.

## Description

This application claims the priority of Chinese patent application No. 200910259272.2 filed with the Chinese Patent Office on December 17, 2009 and entitled "A Total Mixed Anaerobic Reaction Circulation Device and A Circulation Method of Methane Reaction Materials", the disclosure of which is incorporated herein in its entirety by reference.

### Field of the Invention

The present invention relates to a total mixed anaerobic reaction circulation device for the methane that applies very well to the methane production with the crops straw, animal manure and organic waste water as the main materials.

### Background Art

Anaerobic fermentation is widely used in the treatment of the organic substance, for instance, producing methane, treating the organic waste and waste liquid rich in the organic substance.

The methane pool is one of the most original applications of anaerobic fermentation, in which the crops straw and animal manure are used as the main materials to produce the methane for daily life uses.

The prepared methane material is usually added from the top portion of the methane pool. The insufficient mixing easily causes the phenomenon of caking in the top portion, which blocks the generation of the methane.

It is not convenient to arrange the mechanical stirring apparatus in the traditional methane pool. In the process of industrialization, the methane pool is replaced by the anaerobic reaction tank that facilitates the disposition of the mechanical stirring apparatus or mechanism. Most of the stirring apparatuses or mechanisms are vertically arranged in the anaerobic reaction tank. Also, some stirring apparatuses or mechanisms are horizontally arranged in the anaerobic reaction tank or arranged in abnormity.

The stirring not only can prevent the caking in the top portion of the anaerobic reaction tank, but also makes the materials uniformly mixed, which can increase the yield of the methane. However, the realization of the mechanic stirring is also followed by the increase of the energy consumption.

The increase of the energy consumption is not desired by people. Then, reducing the energy consumption accordingly becomes the objective that people are eager to achieve.

As the technical exploration to reduce the energy consumption, the Chinese utility model patent ZL200820123345.6 discloses a horizontal anaerobic reactor for generating methane by the fermentation of the organic waste solid, comprising a horizontal tank with a horizontally arranged stirrer, the horizontal tank being hung on its exterior with a circulation waterpot for controlling the temperature.

The above horizontal tank has to some extent the function in reducing the energy consumption, but still can not avoid the disadvantage of high energy consumption due to the mechanical stirrer.

The Chinese invention patent application 200810063934.4 discloses a methane generating device, in which a down-flow anaerobic filter and an up-flow anaerobic fermentation tank are combined to form a set of methane fermentation equipment, therefore the down-flow anaerobic filter and the up-flow anaerobic fermentation tank not only can independently accomplish the objective of producing the methane, but also can cooperate with each other.

The above down-flow anaerobic filter and the up-flow anaerobic fermentation tank realize respective self-circulation stirring, by means of a common set of circulation line with a transporting pump, and at the same time, the active sludge material liquid and supernatant in the anaerobic fermentation tank can be supplied to the down-flow anaerobic filter, to mix the fermented material liquid backflow with the material liquid newly fed.

The above methane generating device makes profitable exploration in the aspect of reducing the energy consumption. However, the circulation line with the transporting pump is provided with a plurality of valves, such that the operation and control are quite complex. As to the methane generating device, it is mainly used in the agriculture and rural areas and serves peasantry, and then the complex operation and control certainly will restrict the popularization and application of the methane project.

In fact, the down-flow anaerobic filter, with the filling material layer therein, is the common anaerobic processing device for the sewage rich in the organic substance, and is mainly used in the sewage processing field, but it seems to be somewhat "delicate" for the application of the methane reaction materials which mainly comprise the straws and manure.

The Chinese invention patent application 200910064439.X discloses a fermentation tank. The tank wall of the fermentation tank is provided thereon with a circulation pipe communicating with the cavity in the tank. The circulation pipe has the upper port under the liquid level of the fermentation broth and the lower port adjacent to the bottom portion of the fermentation tank. The circulation pipe is provided a circulation pump therein.

The fermentation tank disclosed by the above invention patent application can accelerate the self-circulation stirring of the methane materials by means of the circulation pipe , and greatly simplifies the operational complexity.

During implementing the methane device, the present inventors note that the above methane device still has some inconveniences during the operation. Since the volume of the fermentation tank is too large, it is not convenient to additionally provide heat-exchanging and heating device, then it can only run in the greenhouse or is built where the temperature is appropriate, which troubles the application of the methane device, and can hardly be applicable in the areas such as the north where winter is quite long. And it is not convenient when the PH value of the methane reaction materials in the fermentation tank should be adjusted.

In addition, the present inventors note that the self-circulation of the methane reaction materials still results in quite high level of the energy consumption, and the large resistance of the self-circulation of the methane reaction materials causes severe wear on the pipe and the pump, and at the same time induces the hidden danger of obstructing the pump and the pipe.

Owing to the above, the existing anaerobic filters cannot simultaneously take into consideration the aspects of the methane material circulation, adaptability to the materials, yield of the methane and the energy consumption, and have one or more of the disadvantages of non-uniform circulation of the methane reaction materials, singleness of the material, small volume, low yield of the methane (only 1.0 can be achieved) and high energy consumption.

### Summary of the Invention

The present invention aims at providing a total mixed anaerobic reaction circulation device for methane, which can be adapted to different methane reaction materials, and can save the supplementation of energy and water into the circulation of the reaction materials. The present invention also aims at providing a circulation method of the methane reaction materials.

Therefore, the present invention, in one aspect, provides a total mixed anaerobic reaction circulation device for methane, comprising a total mixed anaerobic reaction tank and a circulation tank, wherein, a top portion of the total mixed anaerobic reaction tank is connected to the circulation tank via an overflow pipe;a lower portion of the circulation tank is connected to a lower portion of the total mixed anaerobic reaction tank via a transporting pipe and a material transporting pump; and the circulation tank is connected to a transporting pipe that supplies methane reaction materials.

Further, the above circulation tank is provided with a heat exchanger therein.

Further, the above circulation tank is provided with a central cylinder therein, wherein the overflow pipe is connected to the central cylinder, and a lower end of the central cylinder has an outlet pipe.

Further, the above central cylinder has a central pipe therein, the central pipe communicates with a feeding pipe and a discharging pipe, respectively.

Further, an inner peripheral wall of the above circulation tank is provided with a flow deflector thereon.

Further, the top side of the above total mixed anaerobic reaction tank is provided with a purge air cylinder, wherein, the overflow pipe is connected to the bottom portion of the purge air cylinder, the total mixed anaerobic reaction tank and the purge air cylinder are connected via a U-shaped pipe.

Further, a lower portion of the above total mixed anaerobic reaction tank at least has two separated feeding ports connected to the transporting pipe.

Further, the above circulation tank has a material transporting pump and a second transporting pipe connected to a lower portion of the total mixed anaerobic reaction tank.

Further, the above total mixed anaerobic reaction tank has a volume of 400 ~6000 m³.

Further, a volume of the above circulation tank is 1/10~1/20 of the volume of the total mixed anaerobic reaction tank.

According to the other aspect of the present invention, the present invention provides a circulation method of methane reaction materials, comprising: creating a total mixed anaerobic reaction tank having a first volume and a circulation tank having a second volume; firstly pumping the methane reaction materials to the circulation tank, then, and then pumping the methane reaction materials from a bottom portion of the circulation tank to a bottom portion of the total mixed anaerobic reaction tank; and overflowing a supernatant into the circulation tank when the total mixed anaerobic reaction tank is full of materials, mixing the supernatant with the methane reaction materials in the circulation tank to be pumped into the total mixed anaerobic reaction tank, so as to use the supernatant in the total mixed anaerobic reaction tank for self-circulation.

Further, the supernatant overflowing into the circulation tank is made to firstly flow into a central cylinder in the circulation tank, and then into a bottom portion of the circulation tank.

In the present invention, the supernatant being used as the working medium for circulation can sufficiently mix the methane reaction materials in the anaerobic reaction tank, so as to improve the yield of the methane. The circulation use of the supernatant can save the water and energy supplementation. Compared with the methane reaction materials being used as the circulation working medium, the flow resistance of the liquid is quite small, thereby the wear on the pipe and pump is reduced; and correspondingly, the requirements on the system are lowered. Compared with the methane being used as the circulation working medium, the security risk brought about by the methane used as the circulation working medium is avoided.

In the present invention, the use of the circulation tank simultaneously takes into account the functions such as addition of the methane reaction materials, heating of the methane reaction materials, adjustment of PH value, methane liquid recovery, etc. The total mixed anaerobic reaction circulation device for methane according to the present invention is suitable for the large-scale industrialized methane project and can be popularized and used in cold areas.

Apart from the objectives, features and advantages mentioned above, the present invention also has some other objectives, features and advantages. The present invention will be further illustrated in detail with reference to the drawings.

### Brief Description of the Accompanying Drawings

The accompanying drawings, constituting a part of the Description for further understanding the present invention, show the preferred embodiments of the present invention, and illustrate the principle of the present invention together with the Description. In the drawings:
Figure 1 is a process flow chart of the total mixed anaerobic reaction circulation device for methane according to a preferred embodiment of the present invention;
Figure 2 is a structural schematic view of the total mixed anaerobic reaction circulation device for methane according to a preferred embodiment of the present invention;
Figure 3 is a top view of the total mixed anaerobic reaction circulation device for methane as shown in Figure 2; and
Figure 4 is a half sectional view of the circulation tank in the total mixed anaerobic reaction circulation device for methane as shown in Figure 2.

### Detailed Description of the Invention

Next, the embodiments of the present invention are illustrated in detail with reference to the drawings, but the present invention can be embodied in various embodiments as defined and covered by the claims.

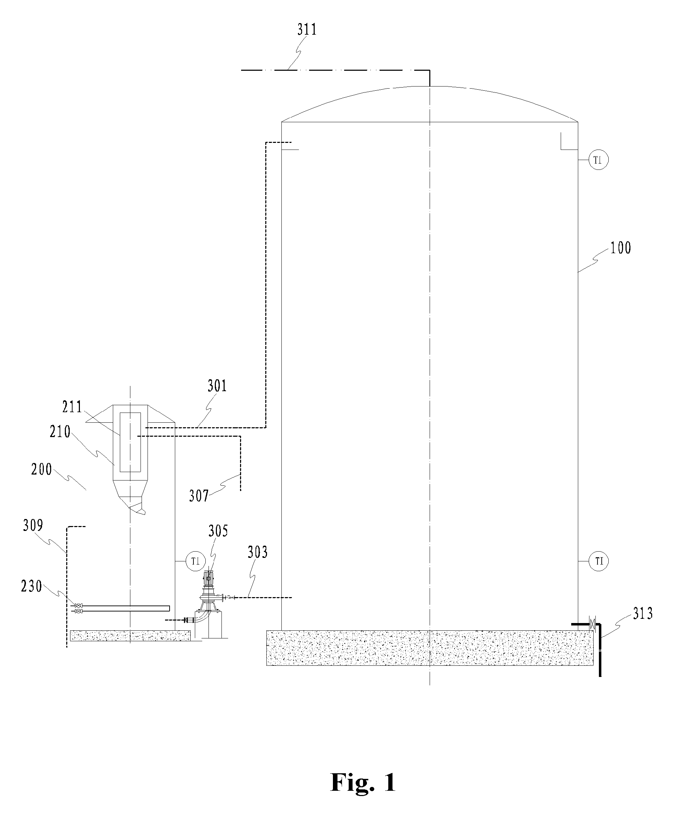

Figure 1 is the process flow chart of the total mixed anaerobic reaction circulation device for methane according to the present invention. As shown in Figure 1, the total mixed anaerobic reaction circulation device comprises the anaerobic reaction tank 100 and the circulation tank 200.

The circulation tank 200 receives the methane reaction materials from the transporting pipe 309. The methane reaction materials can be the mixture of straws, animal manure and organic waste water, for instance, the mixture having the wheat stem and cattle manure as the main materials.

The circulation tank 200, via the material transporting pipe 303 and the transporting pump 305 at its lower portion, pumps the reaction materials in the circulation tank to the lower portion of the total mixed anaerobic reaction tank 100.

The circulation tank 200 is provided with the central cylinder 210 therein. The supernatant in the top portion of the total mixed anaerobic reaction tank 100 is transported into the central cylinder 210 via the overflow pipe 301, then the supernatant flows into the inner cavity of the circulation tank 200 from the central cylinder 210.

The circulation tank 200 is provided with the heat exchanger 230 therein. The heat exchanger 230 can be connected to the heat source such as a vapor source, so as to heat-exchange and heat the reaction materials in the circulation tank 200, to maintain the methane reaction materials in the total mixed anaerobic reaction tank 100 at an appropriate reaction temperature, for instance, between 35□ and 38□.

Inside the housing 211 of the central cylinder 210 of the circulation tank 200 is the central pipe 212 provided wherein the central pipe 212 is connected to the discharging pipe 307 through which the spare methane fluid can be discharged into the methane pool (not shown in the figure).

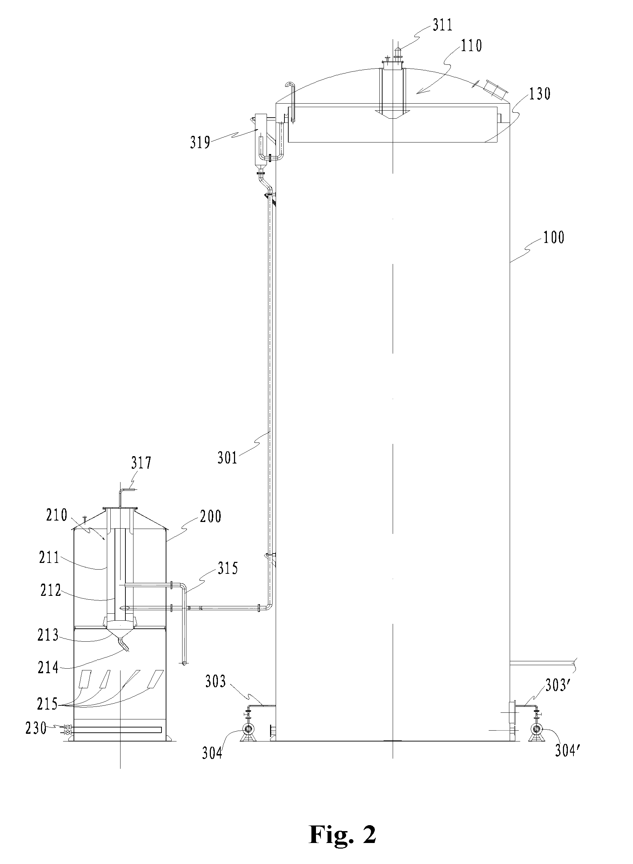

The central cylinder of the circulation tank 200 is further connected with the feeding pipe 315 (shown in Figure 2) to supplement methane fluid or water.

The top portion of the total mixed anaerobic reaction tank 100 has the methane pipe 311 to export the methane generated in the tank, for instance, into the methane storage tank (not shown in the figure). The bottom portion of the total mixed anaerobic reaction tank 100 is provided with the slag-removal pipe 313 through which the fermented methane reaction materials can be removed out of the tank.

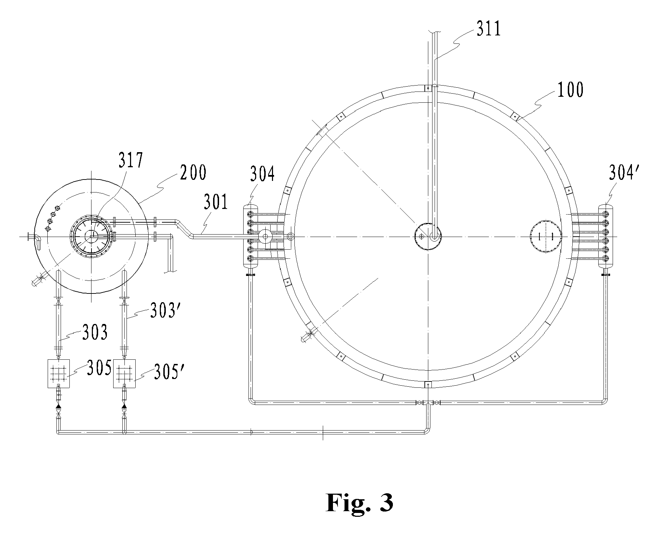

Figure 2 and Figure 3 are the structural schematic view of the total mixed anaerobic reaction circulation device for methane according to the preferred embodiment of the present invention.

The top portion in the total mixed anaerobic reaction tank 100 is provided with the trash barrier cover 130 and the methane collector 110, the trash barrier cover 130 used for filtering the solids of the methane reaction materials in the total mixed anaerobic reaction tank 100.

The methane collector 110 is well-known by one skilled in the art and unnecessary details will not be given.

The supernatant is guided into the purge air cylinder 319 via the U shaped pipe. A small amount of the methane contained in the supernatant can overflow to facilitate the supernatant flowing in the overflow pipe 301. Of course, the supernatant also can be made to directly flow into the circulation tank 200 via the overflow pipe 301.

Both sides of the lower portion of the total mixed anaerobic reaction tank 100 are provided with the general feeding pipes 304 and 304' that are extended into the total mixed anaerobic reaction tank 100 via several branch feeding pipes. The methane reaction materials are pumped to the general feeding pipes 304 and 304' by the transporting pumps 305 and the transporting pipe 303.

Moreover, the methane reaction materials can also be pumped to the general feeding pipes 304 and 304' by the stand-by pump 305' and the transporting pipe 303'.

The methane reaction materials in the tank can be mixed more uniformly by feeding the reaction materials from both sides of the bottom portion of the total mixed anaerobic reaction tank 100. Certainly, more general feeding pipes 304 can be disposed around the tank to eliminate the dead space of the material circulation.

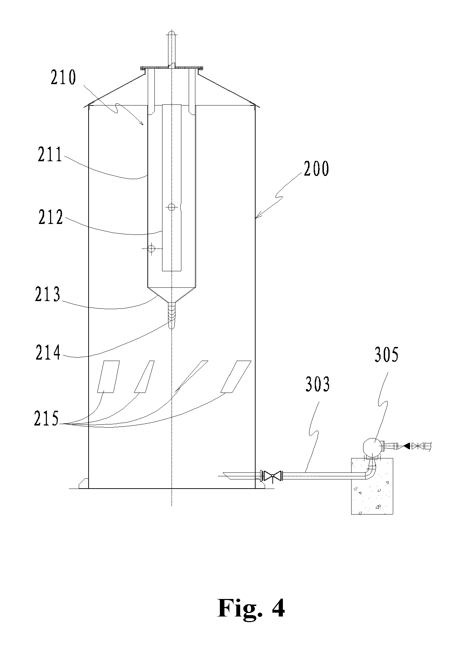

The preferred structure of the circulation tank 200 is schematically shown in Figure 2 to Figure 4.

The circulation tank 200 is provided with the central cylinder 210 therein, which has the functions at least in two aspects: in one aspect, forming a container for temporarily accommodating the supernatant and for separating the supernatant from the methane reaction materials coming from the transporting pipe 309, to facilitate the operations such as adjusting the PH value and using the supernatant; and in the other aspect, making it convenient to dispose the central pipe 212.

The central cylinder 210 is supported at the upper portion of the circulation tank 200, and its lower portion has the bending outlet pipe 214, the outlet port of which faces to the flow deflector plate or swirl plate 215 to create flow swirling, thereby uniformly mixing the materials.

The outlet pipe 214 can be a hose, such that the supernatant or water flowing from the outlet port injects onto the flow deflector plate 215.

Alternatively, the outlet pipe 214 is rotatable around the central axis of the central cylinder 210, and its outlet port has the direction located on the tangential direction of the circumference centering on the central axis, so as to automatically rotate under the counterforce of the water flow.

The top portion of the circulation tank 200 has the air exhaust tube 317 that can guide the methane generated in the circulation tank 200 into the methane storage tank.

Meanwhile, a PH value adjusting port is reserved in the top portion of the circulation tank 200, and certainly, ports for some other purposes also can be reserved.

In one embodiment, the total mixed anaerobic reaction circulation device of the present invention is a large-scale methane generating device, and its methane production ratio can be up to 1.6. Preferably, the circulation tank is a steel-structured atmospheric vessel, with the external dimension of 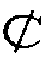 2.6m×6m (H), effective liquid depth of 5.7 m, and effective volume of 30 m³. The total mixed anaerobic reactor is made of carbon steel, characterized by heat preservation and anti-corrosion at the interior and exterior. As for a single set, the dimension is 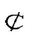 6.6mx16m (H). The effective water depth is 15.7 m, effective volume is 530 m³, and the anaerobic reaction material has the maintaining time of 15 days.

In the other embodiments, the external dimensions and volumes of the total mixed anaerobic reaction tank and the circulation tank can be modified.

The operating process of the device according to the present invention is as follow: the materials firstly are filled into the circulation tank, then pumped by the pump from the bottom portion of the circulation tank into the total mixed anaerobic reaction tank. When the total mixed anaerobic reaction tank is full with the materials, along with the continuous working of the pump, the materials overflow from the top portion of the total mixed anaerobic reaction tank and enter the circulation tank via the pipe. The materials are sufficiently stirred in the continuous circulation in the circulation tank and the total mixed anaerobic reaction tank.

According to the present invention, the circulation is sufficient, the energy consumption is low, the methane yield can reach 1.6, and the materials have the variety.

The above is merely the preferred embodiments of the present invention and not to limit the present invention. For one skilled in the art, the present invention may have various alterations and changes. Any modification, equivalent substitution, improvement, etc., within the spirit and principle of the present invention, should be included in the protection scope of the present invention.

## Claims

1. A total mixed anaerobic reaction circulation device for methane, **characterized by** comprising a total mixed anaerobic reaction tank (100) and a circulation tank (200), wherein
a top portion of the total mixed anaerobic reaction tank (100) is connected to the circulation tank (200) via an overflow pipe (301);
a lower portion of the circulation tank (200) is connected to a lower portion of the total mixed anaerobic reaction tank (100) via a transporting pipe (303) and a material transporting pump (305); and
the circulation tank (200) is connected to a transporting pipe (309) that supplies methane reaction materials.

2. The total mixed anaerobic reaction circulation device for methane according to Claim 1, **characterized in that** the circulation tank (200) is provided with a heat exchanger (230) therein.

3. The total mixed anaerobic reaction circulation device for methane according to Claim 1, **characterized in that** the circulation tank (200) is provided with a central cylinder (210) therein, wherein the overflow pipe (301) is connected to the central cylinder (210), and a lower end of the central cylinder (210) has an outlet pipe (214).

4. The total mixed anaerobic reaction circulation device for methane according to Claim 3, **characterized in that** the central cylinder (210) has a central pipe (212) therein, the central pipe (212) is connected with a feeding pipe (315) and a discharging pipe (307), respectively.

5. The total mixed anaerobic reaction circulation device for methane according to Claim 1, **characterized in that** an inner peripheral wall of the circulation tank (200) is provided with a flow deflector (215) thereon.

6. The total mixed anaerobic reaction circulation device for methane according to Claim 1, **characterized in that** a lower portion of the total mixed anaerobic reaction tank (100) at least has two separated feeding ports connected to the transporting pipe (303).

7. The total mixed anaerobic reaction circulation device for methane according to Claim 1, **characterized in that** the circulation tank (200) has a material transporting pump (305') and a second transporting pipe (303') connected to a lower portion of the total mixed anaerobic reaction tank (100).

8. The total mixed anaerobic reaction circulation device for methane according to Claim 1, **characterized in that** the total mixed anaerobic reaction tank (100) has a volume of 400 -6000 m³, and a volume of the circulation tank (200) is 1/10~1/20 of the volume of the total mixed anaerobic reaction tank (100).

9. A circulation method of methane reaction materials, **characterized by** comprising:
providing a total mixed anaerobic reaction tank (100) having a first volume and a circulation tank (200) having a second volume;
firstly pumping the methane reaction materials to the circulation tank (200), then, and then pumping the methane reaction materials from a bottom portion of the circulation tank (200) to a bottom portion of the total mixed anaerobic reaction tank (100); and
overflowing a supernatant into the circulation tank (200) when the total mixed anaerobic reaction tank (100) is full of materials, mixing the supernatant with the methane reaction materials in the circulation tank (200) to be pumped into the total mixed anaerobic reaction tank (100), so as to make use of the supernatant in the total mixed anaerobic reaction tank (100) for self-circulation.

10. The circulation method of methane reaction materials according to Claim 9, **characterized in that** the supernatant overflowing into the circulation tank (200) is firstly flowed into a entral cylinder (210) of the circulation tank (200), and then into a bottom portion of the circulation tank (200).
